# EUROPEAN PATENT SPECIFICATION

(11) **EP 2 601 944 B1**
(45) Date of publication and mention of the grant of the patent: **17.02.2021**
(21) Application number: 11814519.2
(22) Date of filing: 27.07.2011
(51) Int. Cl.: A61K 31/137, A61K 9/70, A61K 47/32, A61P 13/10

(54) **TRANSDERMAL PATCH AND METHOD FOR AUGMENTING ADHESIVE STRENGTH THEREOF**
TRANSDERMALES PFLASTER UND VERFAHREN ZUR ERHÖHUNG VON DESSEN HAFTFESTIGKEIT
TIMBRE TRANDERMIQUE ET PROCÉDÉ PERMETTANT D'AUGMENTER SON ADHÉRENCE

(30) Priority: 03.08.2010 JP 2010174157
(43) Date of publication of application: 12.06.2013
(73) Proprietor: Hisamitsu Pharmaceutical Co., Inc., Tosu-shi, Saga 841-0017 (JP)
(72) Inventor: SHINODA Tomohiro, Tsukuba-shi Ibaraki 305-0856 (JP); UCHIDA Naoyuki, Tsukuba-shi Ibaraki 305-0856 (JP); TAKADA Yasunori, Tsukuba-shi Ibaraki 305-0856 (JP)
(74) Representative: Grünecker Patent- und Rechtsanwälte PartG mbB
(86) International application number: PCT/JP2011/067094
(87) International publication number: WO 2012/017892

(56) References cited:
- EP-A1- 1 844 773
- WO-A1-00/12069
- WO-A1-2009/146443
- DE-A1- 19 932 651
- DE-U1- 29 923 242
- JP-A- 2002 544 222
- JP-A- 2010 215 658
- US-B1- 6 517 864

## Description

### [Technical Field]

The present invention relates to a patch and a method for enhancing adhesion force of a patch.

### [Background Art]

Conventionally, patches for transdermally administrating active ingredients have been developed from the viewpoints of reducing adverse effects associated with oral administration and of improving the quality of life of patients. Patches for transdermal administration are disclosed also for tolterodine (4-methyl-2-[(R)-3-(diisopropylamino)-1-phenylpropyl] phenol), which is an active ingredient for treating urinary urgency, urinary incontinence, and the like, and tolterodine derivatives.

For example, International Application Japanese-Phase Publication No. 2002-523446 (PTL 1) describes a transdermal patch for transdermally administering salts, prodrugs, and metabolites of tolterodine. Meanwhile, International Application Japanese-Phase Publication No. 2002-544222 (PTL 2) describes a transdermal therapeutic system (TTS) comprising a (meth) acrylate copolymer containing ammonium groups, at least one plasticizer, and up to 25% by mass tolterodine. Moreover, International Application Japanese-Phase Publication No. 2006-522759 (PTL 3) describes a device for transdermal delivery of (R)-2-[3-(1,1-diisopropylamino)-1-phenylpropyl]-4-(hyd roxymethyl)phenyl isobutyrate (fesoterodine), which is a 3,3-diphenylpropylamine derivative. However, the transdermal patch described in PTL 1, the transdermal therapeutic system (TTS) described in PTL 2, and the device described in PTL 3 are still insufficient in terms of adhesion force for a patch which is, in general, continuously attached to the skin for a long period. US 6,517,864 B1 discloses a device for transdermal administration comprising at least one compound having a therapeutic effect against an overactive bladder in a living body which is selected from the group consisting of (R) - tolterodine or the racemated therof, salts thereof, prodrugs thereof, and metabolites thereof.
WO 00/12069 A1 relates to a pharmaceutical formulation containing tolterodine or a tolterodine-related compound, or a pharmaceutically accetptable salt thereof, which formulation when administered to a patient provides controlled release of tolterodine or said tolterodine-related compound, or salt thereof, such that a substantially constant serum level of the active moiety or moieties is maintained for at least 24 hours. DE 19932651A1 discloses a transdermal therapeutical device with an amount of tolterodin, it pharmaceutically acceptable salts, derivatives or mixtures thereof. Preferably, the device is in form of a patch with an impermeable top coating and a peelable protective layer. A percutaneous absorption type pharmaceutical preparation is disclosed in EP 1844773 A1 which comprises 4-(2-methyl-1-imidazolyl)-2,2-diphenylbutylamide as the active ingredient and a backbone for transdermal system wherein the content of the active ingredient is from 0.1 mg to 10 mg.
WO 2012/017892 A1 describes a transdermal patch provided with a backing and a pressure-sensitive adhesive layer, wherein said pressure-sensitive adhesive layer includes at least one component selected from the groups consisting of tolterodine acetate and salts thereof.

### [Citation List]

### [Patent Literature]

[PTL 1] International Application Japanese-Phase Publication No. 2002-523446
[PTL 2] International Application Japanese-Phase Publication No. 2002-544222
[PTL 3] International Application Japanese-Phase Publication No. 2006-522759

### [Summary of Invention]

### [Technical Problem]

The present invention has been made in view of the problem of the conventional technologies, and an object of the present invention is to provide a patch having a strong adhesion force to the skin, and a method for enhancing adhesion force of a patch.

### [Solution to Problem]

The present inventors have conducted earnest study to achieve the above object. As a result, the present inventors have found that the adhesion force of a patch is improved when at least one selected from the group consisting of tolterodine acetate, which is an acetylated product of tolterodine, and salts thereof is introduced in advance into an adhesive agent layer of the patch, or is caused to be formed over time in the adhesive agent layer. This finding has led to the completion of the present invention.

Specifically,
a patch of the present invention is a patch comprising a support and an adhesive agent layer, wherein the adhesive agent layer comprises : at least one selected from the group consisting of tolterodine and salts thereof; a (meth)acrylate copolymer containing vinyl acetate as a constituent; and at least one selected from the group consisting of tolterodine acetate and salts thereof derived from the at least one selected from the group consisting of tolterodine and salts thereof.

In the patch of the present invention, a total content of the tolterodine acetate and the salts thereof is preferably 0.01 to 50% by mass relative to a total mass of the adhesive agent layer, and a total content of the tolterodine and the salts thereof is preferably 3 to 60% by mass relative to the total mass of the adhesive agent layer, and furthermore, a content of the (meth)acrylate copolymer containing vinyl acetate as a constituent is preferably 5 to 95% by mass relative to the total mass of the adhesive agent layer.

Moreover, a method for enhancing adhesion force of a patch of the present invention is a method for enhancing adhesion force of a patch comprising a support and an adhesive agent layer, the method comprising causing at least one selected from the group consisting of tolterodine acetate and salts thereof to be present in the adhesive agent layer. In the method for enhancing adhesion force of a patch of the present invention, the at least one selected from the group consisting of tolterodine acetate and salts thereof is caused to be present, preferably such that a total content of the tolterodine acetate and the salts thereof is 0.01 to 50% by mass relative to a total mass of the adhesive agent layer.

Note that, although it is not exactly clear why the above-described object can be achieved by the patch of the present invention, the present inventors speculate as follows. Specifically, in the patch of the present invention, tolterodine acetate, which is an acetylated product of tolterodine, is contained in the adhesive agent layer according to the present invention. The present inventors speculate that plasticity is hence imparted to the adhesive agent layer, and the adhesion force of the patch is therefore improved.

Moreover, in a case of conventional patches containing only tolterodine, tolterodine is inactivated in a short period by metabolism in the body, because tolterodine itself is directly absorbed transdermally. In contrast, the tolterodine acetate contained in the adhesive agent layer according to the present invention can be converted to tolterodine in the body by deacetylation after transdermal absorption. The present inventors speculate that a blood concentration of tolterodine can be hence maintained for a long period at a therapeutically effective level, and consequently, the patch of the present invention makes it possible to obtain a more persistent effect of tolterodine than the conventional patches containing only tolterodine.

### [Advantageous Effects of Invention]

The present invention makes it possible to provide a patch having a strong adhesion force to the skin, and a method for enhancing adhesion force of a patch.

### [Description of Embodiments]

Hereinafter, the present invention will be described in detail based on preferred embodiments thereof.

A patch of the present invention is a patch comprising a support and an adhesive agent layer, wherein the adhesive agent layer comprises: at least one selected from the group consisting of tolterodine and salts thereof; a (meth)acrylate copolymer containing vinyl acetate as a constituent; and at least one selected from the group consisting of tolterodine acetate and salts thereof derived from the at least one selected from the group consisting of tolterodine and salts thereof.

The support according to the present invention is not particularly limited, and may be stretchable or non-stretchable. Examples of the support include plastic films, woven fabrics, and nonwoven fabrics. Examples of materials of the plastic films, the woven fabrics, and the nonwoven fabrics include polyurethane, polyester, polyvinyl acetate, polyvinylidenechloride, polyethylene, polyethylene terephthalate, aluminum, and composite materials thereof. A thickness of such a support is not particularly limited, and is preferably about 2 to 3000 µm, in general.

The adhesive agent layer according to the present invention comprises at least: at least one selected from the group consisting of tolterodine acetate and salts thereof; an adhesive agent having adhesive property; and an active ingredient . A thickness of such an adhesive agent layer is not particularly limited, and is preferably about 10 to 300 µm, in general.

The tolterodine acetate is an acetylated product of tolterodine
(4-methyl-2-[(R)-3-(diisopropylamino)-1-phenylpropyl]p henol), and is (R)-N,N-diisopropyl-3-(2-ethanoyloxy-5-methylphenyl) -3 -phenylpropanamine in the present invention. The salts of tolterodine acetate are not particularly limited, as long as the salts are pharmaceutically acceptable salts. Examples of the salts include pharmaceutically acceptable acid addition salts. Examples of the acid addition salts include hydrochloric acid salt, hydrobromic acid salt, nitric acid salt, sulfuric acid salt, phosphoric acid salt, formic acid salt, acetic acid salt, trifluoroacetic acid salt, propionic acid salt, lactic acid salt, tartaric acid salt, oxalic acid salt, fumaric acid salt, maleic acid salt, citric acid salt, malonic acid salt, and methanesulfonic acid salt.

In the patch of the present invention, even when tolterodine acetate or a salt thereof is not introduced in advance during the production of the patch, tolterodine acetate or a salt thereof is derived over time from tolterodine or a salt thereof in the adhesive agent layer. Hence, the adhesive agent layer comprises at least one selected from the group consisting of tolterodine acetate and salts thereof formed over time in such a manner. Moreover, the adhesive agent layer may further comprise tolterodine acetate, a salt of tolterodine acetate, or a mixture thereof, which is introduced in advance into the adhesive agent layer.

A total content of the tolterodine acetate and the salts thereof is preferably 0.01 to 50% by mass, and more preferably 0.05 to 10% by mass in the adhesive agent layer. If the content is less than the lower limit, an effect of enhancing adhesion force tends not to be exhibited. Meanwhile, if the content exceeds the upper limit, the adhesion force tends to be excessively enhanced.

The adhesive agent only needs to have self-adhesion force, and is not particularly limited. The adhesive agent preferably comprises a hydrophobic polymer. The hydrophobic polymer is preferably a (meth)acrylic-based polymer, a rubber-based polymer, or a silicone-based polymer.

Such a (meth) acrylic-based polymer may be one obtained by copolymerization using, as a constituent, at least one of (meth)acrylic acid derivatives represented by 2-ethylhexyl acrylate, methyl acrylate, butyl acrylate, hydroxyethyl acrylate, 2-ethylhexyl methacrylate, and the like. Examples of the (meth) acrylic-basedpolymer include acrylic acid-acrylic acid octyl ester copolymers, 2-ethylhexyl acrylate-vinylpyrrolidone copolymers, acrylic acid ester·vinyl acetate copolymers, 2-ethylhexyl acrylate·2-ethylhexyl methacrylate·dodecyl methacrylate copolymers, methyl acrylate·2-ethylhexyl acrylate copolymers, and acrylic resin alkanolamines.

In the patch of the present invention, the adhesive agent needs to comprise, among the (meth)acrylic-based polymers, a (meth)acrylate copolymer containing vinyl acetate as a constituent monomer from the viewpoint that the at least one selected from the group consisting of tolterodine acetate and salts thereof is formed over time in the adhesive agent layer. Examples of such a (meth)acrylate copolymer containing vinyl acetate as a constituent monomer include the above-described acrylic acid ester·vinyl acetate copolymers, and the like. A content of the (meth) acrylate copolymer containing vinyl acetate as a constituent monomer is preferably 5 to 95% by mass, and more preferably 20 to 85% by mass in the adhesive agent layer. If the content is less than the lower limit, the tolterodine acetate tends to be formed insufficiently. Meanwhile, if the content exceeds the upper limit, the tolterodine acetate tends to be formed excessively.

Note that adhesive agents containing the (meth)acrylic-based polymer are available as commercial products. For example, DURO-TAK acrylic adhesive agent series (manufactured by Henkel AG & Co. KGaA), EUDRAGIT series (manufactured by HIGUCHI INC.), GELVA (manufactured by Monsanto Company), MAS (manufactured by Sekisui Chemical Co., Ltd.), and the like can be used.

Examples of the rubber-based polymer include styrene-isoprene-styrene block copolymers, isoprene, polyisobutylene, styrene-butadiene-styrene block copolymers, styrene-butadiene copolymers, and polysiloxanes. Of these rubber-based polymers, polyisobutylene and styrene-isoprene-styrene block copolymers are preferable, and styrene-isoprene-styrene block copolymers are particularly preferable.

Examples of the silicone-based polymer include polydimethylsiloxanes, which are available as commercial products. For example, BIO-PSA series (manufactured by Dow Corning Corporation) and the like can be used.

One of these hydrophobic polymers may be used alone, or two or more thereof may be used in combination. A content of the hydrophobic polymer in the adhesive agent layer according to the present invention is preferably 5 to 95% by mass, more preferably 20 to 85% by mass, and particularly preferably 30 to 75% by mass in the adhesive agent layer. If the content of the hydrophobic polymer is less than the lower limit, the adhesive agent layer tends to be formed insufficiently. Meanwhile, if the content of the hydrophobic polymer exceeds the upper limit, the permeability of the active ingredient tends to decrease.

The active ingredient is transdermally absorbed by means of the patch of the present invention. The active ingredient is not particularly limited, and any active ingredient can be selected depending on the purpose of the treatment in which the patch is used. An active ingredient can be used alone, or two or more active ingredients may be used in combination. In the second patch of the present invention, the active ingredient needs to be any one selected from the group consisting of tolterodine and salts thereof, from the viewpoint that the at least one selected from the group consisting of tolterodine acetate and salts thereof is formed over time in the adhesive agent layer. The salts of tolterodine are not particularly limited, as long as the salts are pharmaceutically acceptable salts. Examples of the salts of tolterodine include pharmaceutically acceptable acid addition salts. Examples of the acid addition salts include acid addition salts similar to those listed as the acid addition salts of tolterodine acetate. A total content of such tolterodine and salts thereof is preferably 3 to 60% by mass, more preferably 10 to 50% by mass, and particularly preferably 15 to 45% by mass in the adhesive agent layer. If the content is less than the lower limit, the tolterodine acetate tends to be formed insufficiently. Meanwhile, if the content exceeds the upper limit, the adhesive agent layer tends to be formed insufficiently, because the content of the adhesive agent is low.

Moreover, the active ingredient in the patch of the present invention is preferably any one selected from the group consisting of tolterodine and salts thereof, also from the viewpoint that the at least one selected from the group consisting of tolterodine acetate and salts thereof is converted after transdermal absorption to tolterodine by deacetylation in the body, which makes it possible to maintain a blood concentration of tolterodine at a therapeutically effective level for a long period, and consequently makes it possible to obtain persistent effect of tolterodine for a long period.

The adhesive agent layer according to the present invention may further comprise an organic acid, a pharmaceutically acceptable salt of the organic acid, an absorption enhancer, and a plasticizer.

The organic acid is not particularly limited, and examples thereof include aliphatic (mono-, di-, or tri-) carboxylic acids such as acetic acid, propionic acid, isobutyric acid, caproic acid, caprylic acid, lactic acid, maleic acid, pyruvic acid, oxalic acid, succinic acid, and tartaric acid; aromatic carboxylic acids such as phthalic acid, salicylic acid, benzoic acid, and acetylsalicylic acid; alkyl sulfonic acids such as methanesulfonic acid, ethanesulfonic acid, propylsulfonic acid, butanesulfonic acid, and polyoxyethylene alkyl ether sulfonic acids; alkyl sulfonic acid derivatives such as N-2-hydroxyethylpiperidine-N'-2-ethanesulfonic acid; and cholic acid derivatives such as dehydrocholic acid. Of these organic acids, acetic acid, propionic acid, lactic acid, or salicylic acid is preferable, and acetic acid is more preferable as the organic acid.

The pharmaceutically acceptable salt of the organic acid may be an inorganic salt or an organic salt. The pharmaceutically acceptable salt is preferably an acetic acid salt, a propionic acid salt, a butyric acid salt, a lactic acid salt, a benzoic acid salt, or a salicylic acid salt, and particularly preferably sodium acetate.

One of these organic acids and salts thereof may be used alone, or two or more thereof may be used in combination. A mixture of the organic acid and the salt thereof may also be used. When at least one selected from the group consisting of such organic acids and salts thereof is contained in the adhesive agent layer according to the present invention, a total content thereof is preferably 0.01 to 30% by mass, more preferably 0.1 to 25% by mass, and particularly preferably 0.1 to 20% by mass in the adhesive agent layer. If the content is less than the lower limit, an amount of the active ingredient transdermally permeated tends to decrease. Meanwhile, if content exceeds the upper limit, irritation to the skin tends to increase.

The absorption enhancer is not particularly limited, and a conventional absorption enhancer known to have an enhancing effect of absorption through the skin can be used as appropriate. Examples of the absorption enhancer include fatty acids having 6 to 20 carbon chains, fatty alcohols, fatty acid esters, amides, ethers, aromatic-based organic acids, aromatic-based alcohols, aromatic-based organic acid esters, aromatic-based organic acid ethers (the above-described absorption enhancers may be saturated or unsaturated, and may be cyclic, linear, or branched), lactic acid esters, acetic acid esters, monoterpene-based compounds, sesquiterpene-based compounds, Azone, Azone derivatives, pirotiodecane, glycerin fatty acid esters, propylene glycol fatty acid esters, sorbitan fatty acid esters (Span-series), polysorbate series (Tween series), polyethylene glycol fatty acid esters, polyoxyethylene hardened castor oil (HCO series), polyoxyethylene alkyl ethers, sucrose fatty acid esters, and vegetable oils.

Of these absorption enhancers, preferably used are caprylic acid, capric acid, caproic acid, lauric acid, myristic acid, palmitic acid, stearic acid, isostearic acid, oleic acid, linoleic acid, linolenic acid, lauryl alcohol, myristyl alcohol, oleyl alcohol, isostearyl alcohol, cetyl alcohol, methyl laurate, hexyl laurate, lauric acid diethanolamide, isopropyl myristate, myristyl myristate, octyldodecyl myristate, cetyl palmitate, salicylic acid, methyl salicylate, salicylic acid ethylene glycol, cinnamic acid, methyl cinnamate, cresol, cetyl lactate, lauryl lactate, ethyl acetate, propyl acetate, geraniol, thymol, eugenol, terpineol, 1-menthol, borneol, d-limonene, isoeugenol, isoborneol, nerol, dl-camphor, glycerin monocaprylate, glycerin monocaprate, glycerin monolaurate, glycerin monooleate, sorbitan monolaurate, sucrose monolaurate, polysorbate 20, propylene glycol, propylene glycol monolaurate, polyethylene glycol monolaurate, polyethylene glycol monostearate, polyoxyethylene lauryl ether, HCO-60, pirotiodecane, and olive oil, and more preferably used are lauryl alcohol, isostearyl alcohol, lauric acid diethanolamide, glycerin monocaprylate, glycerin monocaprate, glycerin monooleate, sorbitan monolaurate, propylene glycol monolaurate, polyoxyethylene lauryl ether, and pirotiodecane. In addition, one of these absorption enhancers may be used alone, or two or more thereof may be used in combination.

When the absorption enhancer is contained in the adhesive agent layer according to the present invention, a content of the absorption enhancer is preferably 0.01 to 20% by mass, more preferably 0.05 to 10% by mass, and particularly preferably 0.1 to 5% by mass in the adhesive agent layer. If the content of the absorption enhancer is less than the lower limit, the amount of the active ingredient transdermally permeated tends to decrease. Meanwhile, if the content of the absorption enhancer exceeds the upper limit, irritation to the skin tends to increase, so that redness, edema, or the like occurs.

Examples of the plasticizer include petroleum-based oils such as paraffin-based process oil, naphthene-based process oil, and aromatic-based process oil; squalane; squalene; vegetable-based oils such as olive oil, camellia oil, castor oil, tall oil, and peanut oil; silicone oils; dibasic acid esters such as dibutyl phthalate and dioctyl phthalate; liquid rubbers such as liquid polybutene and liquid isoprene rubber; liquid fatty acid esters such as isopropyl myristate, hexyl laurate, diethyl sebacate, and diisopropyl sebacate; diethylene glycol; polyethylene glycol; glycol salicylate; propylene glycol; dipropylene glycol; triacetin; triethyl citrate; and crotamiton. Of these plasticizers, liquid paraffin, liquid polybutene, isopropyl myristate, diethyl sebacate, and hexyl laurate are particularly preferable. In addition, one of these plasticizers may be used alone, or two or more thereof may be used in combination.

When such a plasticizer is contained in the adhesive agent layer according to the present invention, a content of the plasticizer is preferably 10 to 70% by mass, more preferably 10 to 60% by mass, and particularly preferably 10 to 50% by mass in the adhesive agent layer. If the content of the plasticizer is less than the lower limit, a cohesive force sufficient for a patch tends not to be maintained. Meanwhile, if the content of the plasticizer exceeds the upper limit, the permeability of the active ingredient tends to be lowered.

Moreover, the adhesive agent layer according to the present invention may further comprise a tackifier resin, from the viewpoint of further improvement of the adhesion force. Examples of the tackifier resin include rosin and rosin derivatives such as rosin glycerin ester, hydrogenated rosin, hydrogenated rosin glycerin ester, and rosin pentaerythritol ester; alicyclic saturated hydrocarbon resins (for example, "ARKON P100" manufactured by Arakawa Chemical Industries, Ltd.); aliphatic-based hydrocarbon resins (for example, "Quintone B170" manufactured by Zeon Corporation); terpene resins (for example, "Clearon P-125" manufactured by YASUHARA CHEMICAL CO., LTD); and maleic acid resins. The tackifier resin is particularly preferably a hydrogenated rosin glycerin ester, an alicyclic saturated hydrocarbon resin, an aliphatic-based hydrocarbon resin, or a terpene resin.

When such a tackifier resin is contained in the adhesive agent layer according to the present invention, a content of the tackifier resin is preferably 5 to 70% by mass, more preferably 5 to 60% by mass, and particularly preferably 10 to 50% by mass in the adhesive agent layer. If the content of the tackifier resin is less than the lower limit, a sufficient adhesion force tends not to be obtained. Meanwhile, if the content of the tackifier resin exceeds the upper limit, irritation to the skin tends to increase at the time of peeling.

Moreover, if necessary, the adhesive agent layer according to the present invention may further comprise additives such as an antioxidant, a filler, across-linking agent, a preservative, and an ultraviolet absorber. The antioxidant is preferably tocopherol, an ester derivative thereof, ascorbic acid, ascorbyl stearate, nordihydroguaiaretic acid, dibutylhydroxytoluene, or butylhydroxyanisole. The filler is preferably calcium carbonate; magnesium carbonate; a silicate such as aluminum silicate or magnesium silicate; silicic acid; barium sulfate; calcium sulfate; calcium zincate; zinc oxide; or titanium oxide. The cross-linking agent is preferably a thermosetting resin such as an amino resin, a phenolic resin, an epoxy resin, an alkyd resin, or an unsaturated polyester, an isocyanate compound, a block isocyanate compound, an organic-based cross-linking agent, or an inorganic-based cross-linking agent such as a metal or a metal compound. The preservative is preferably ethylparaben, propyl paraben, or butyl paraben. The ultraviolet absorber is preferably a p-aminobenzoic acid derivative, an anthranilic acid derivative, a salicylic acid derivative, acoumarin derivative, an amino acid-based compound, an imidazoline derivative, a pyrimidine derivative, or a dioxane derivative.

When such additives are contained in the adhesive agent layer according to the present invention, a content of the additives is preferably 10% by mass or less, more preferably 7.5% by mass or less, and particularly preferably 5% by mass or less in the adhesive agent layer.

Moreover, the patch of the present invention may further comprise a release liner, in addition to the support and the adhesive agent layer. Such a release liner protects the adhesive agent layer, and protects the active ingredient from degradation, until the patch is applied to the skin. The release liner is preferably coated with silicone so as to enable easy release. Examples of the release liner include films made of polyethylene, polyethylene terephthalate, or polypropylene and coated with silicone.

A method for producing the patch of the present invention is not particularly limited, and a known method for producing a patch can be employed as appropriate. For example, a method may be employed in which the adhesive agent layer is formed by spreading an adhesive agent layer composition comprising the active ingredient and the adhesive agent on the support to obtain the patch of the present invention comprising the support and the adhesive agent layer.

In a method for producing the patch of the present invention, the adhesive agent layer composition comprises at least one selected from the group consisting of tolterodine and salts thereof as the active ingredient, and a (meth)acrylate copolymer containing vinyl acetate as a constituent as the adhesive agent. Moreover, in the second patch of the present invention, it is not necessary to introduce tolterodine acetate or a salt thereof into the adhesive agent layer composition in advance, because at least one selected from the group consisting of tolterodine acetate and salts thereof is formed over time in the adhesive agent layer according to the present invention. However, the adhesive agent layer composition may comprise the tolterodine acetate and the salts of tolterodine acetate, and may further comprise the organic acid, the pharmaceutically acceptable salt of an organic acid, the absorption enhancer, the plasticizer, the tackifier resin, and the additives.

A method for obtaining the adhesive agent layer composition is not particularly limited, and examples thereof include a method (a hot-melt method) in which the materials of the adhesive agent layer composition are thermally melt with mixing, or a method (a solvent method) in which the materials are dissolved in a solvent such as toluene, hexane, or ethyl acetate. When the hot-melt method is employed, the obtained adhesive agent layer composition is spread in a predetermined thickness on the support, and then cooled at room temperature. Thus, the adhesive agent layer formed on the support can be obtained. When the solvent method is employed, the obtained adhesive agent layer composition is spread in a predetermined thickness on the support, and then the solvent is removed by drying. Thus, the adhesive agent layer can be obtained.

Moreover, when the patch of the present invention comprises the release liner, the obtained adhesive agent layer and the release liner are laminated on each other. Thus, a patch of the present invention can be obtained which comprises the support, the adhesive agent layer, and the release liner. Alternatively, the patch of the present invention can also be obtained by spreading the adhesive agent layer composition on the release liner to form an adhesive agent layer, and laminating the obtained adhesive agent layer and the support on each other.

The method for producing the patch of the present invention needs to include a step of causing at least one selected from the group consisting of tolterodine acetate and salts thereof to be formed over time in the adhesive agent layer, after formation of the adhesive agent layer. The step may be a method in which the obtained patch is allowed to stand at a temperature of 0 to 100°C, preferably 20 to 80°C for a certain period. The standing time is preferably 7 days to 3 years, and more preferably 7 days to a half year. If the standing time is less than the lower limit, the tolterodine acetate tends to be formed insufficiently. Meanwhile, if the standing time exceeds the upper limit, the tolterodine acetate tends to be formed excessively.

Next, a method for enhancing adhesion force of a patch of the present invention is described. The method for enhancing adhesion force of a patch of the present invention is a method for enhancing adhesion force of a patch comprising a support and an adhesive agent layer, the method comprising causing at least one selected from the group consisting of tolterodine acetate and salts thereof to be present in the adhesive agent layer.

The support and the adhesive agent layer are not particularly limited. Examples of each of the support and the adhesive agent layer are the same as those shown as the examples of the support or the adhesive agent layer of the patch of the present invention. In addition, examples of the tolterodine acetate and the salts thereof are the same as those shown as the examples of tolterodine acetate and salts thereof in the patch of the present invention. Moreover, the method for enhancing adhesion force of a patch of the present invention is preferably such that a total content of the tolterodine acetate and the salts thereof is 0.01 to 50% by mass relative to a total mass of the adhesive agent layer.

In the method for enhancing adhesion force of a patch of the present invention, a method for causing at least one selected from the group consisting of tolterodine acetate and salts thereof to be present in the adhesive agent layer is not particularly limited, and may be a method in which the at least one selected from the group consisting of tolterodine acetate and salts thereof is introduced into the adhesive agent layer in advance, a method in which the at least one selected from the group consisting of tolterodine acetate and salts thereof is caused to be formed over time in the adhesive agent layer, or a method in which these methods are combined.

The method in which the at least one selected from the group consisting of tolterodine acetate and salts thereof is introduced in advance may be, for example, a method in which the at least one selected from the group consisting of tolterodine acetate and salts thereof is mixed with the adhesive agent layer composition during the production of the patch. Regarding the method in which the at least one selected from the group consisting of tolterodine acetate and salts thereof is caused to be formed over time, the patch at least needs to be produced such that the adhesive agent layer comprises at least one selected from the group consisting of tolterodine and salts thereof; and the (meth) acrylate copolymer containing vinyl acetate as a constituent, from the viewpoint that the at least one selected from the group consisting of tolterodine acetate and salts thereof is formed over time in the adhesive agent layer by acetylation of tolterodine or a salt thereof. The method in which these methods are combined may be, for example, a method in which at least one selected from the group consisting of tolterodine and salts thereof, the (meth)acrylate copolymer containing vinyl acetate as a constituent, and at least one selected from the group consisting of tolterodine acetate and salts thereof are mixed in the adhesive agent layer composition during the production of the patch. A method for producing the patch is not particularly limited, and a known method for producing a patch can be employed as appropriate. For example, it is possible to employ the same methods as those shown as the examples of the method for producing the patch of the present invention.

### [Examples]

Hereinafter, the present invention will be described more specifically based on Examples and Comparative Examples.

### (Example 1)

First, tolterodinetartrate (0.9g), sodium hydroxide (0.15 g: 2 molar equivalents to the tolterodine tartrate), and tolterodine acetate (0.026 g) were added to an ethyl acetatesolution (solid content concentration: 40% by mass) of an acrylic adhesive agent (4.97 g: "DURO-TAK 4287" manufactured by Henkel AG & Co. KGaA). Thus, an adhesive agent layer composition was obtained.

Subsequently, an adhesive agent layer was formed by applying the obtained adhesive agent layer composition to a thickness of 100 µm onto a PET film ("Scotchpak 9732" manufactured by 3M Company). Thus, a patch was obtained into which 3 mg/cm² (30% by mass in the adhesive agent layer) of tolterodine tartrate and 0.085 mg/c² (0.85% by mass in the adhesive agent layer) of tolterodine acetate were incorporated.

### (Comparative Example 1)

A patch was obtained in the same manner as in Example 1, except that no tolterodine acetate was added.

### (Example 2)

A target patch was obtained by allowing a patch to stand at a temperature of 60°C for 2 weeks, the patch being obtained in the same manner as in Example 1, except that no tolterodine acetate was added.

### (Comparative Example 2)

A patch was obtained in the same manner as in Example 2, except that an ethyl acetate solution of an acrylic adhesive agent (5.07 g: "MAS 811" manufactured by Sekisui Chemical Co., Ltd.) which did not contain vinyl acetate as a constituent monomer was used as the acrylic adhesive agent.

### (Comparative Example 3)

A patch was obtained in the same manner as in Example 2, except that 0.75 g of tolterodine tartrate was used, that 0.13 g of sodium hydroxide was used, that a toluene solution containing 0.69 g of a styrene-isoprene-styrene block copolymer and 0.29 g of polyisobutylene was used instead of the ethyl acetate solution of the acrylic adhesive agent, and that 1.79 g of a tackifier resin ("ARKON P100") and 1.34 g of liquid paraffin were added.

### <Peeling Test>

The patches obtained in Example 1 and Comparative Example 1 were each subjected to a peeling test within 48 hours after the patch was obtained. The peeling test was carried out in accordance with Method 1 of JIS Z 0237 "Testing methods of pressure-sensitive adhesive tapes and sheets," and the adhesion force of each patch was measured by using a tensile tester ("TENSILON RTM-100" manufactured by Orientec Co., Ltd.). Table 1 shows the obtained results.

**[Table 1]**

| Sample | Tolterodine acetate (mg/cm²) | Adhesion force (N/cm) |
|---|---|---|
| Example 1 | 0.085 | 7.21 |
| Comp. Ex. 1 | 0 | 6.60 |

### <Evaluation of Tolterodine Acetate Formation>

A 6.25-cm² sample was taken from each of the patches obtained in Example 2 and Comparative Examples 1 to 3, and a drug extraction liquid was obtained by adding 20 g of methanol to the sample, followed by stirring at room temperature for 5 hours. Tolterodine acetate in the obtained extraction liquid was detected by using a high performance liquid chromatograph (manufactured by Shimadzu Corporation, column: ODS column, solvent: phosphate buffer (0.2 mass/volume %)/methanol=50/50 (volume ratio), detection wavelength: UV 210 nm). Thus, formation of tolterodine acetate in each sample was evaluated. Note that the patch obtained in Comparative Example 1 was subjected to this measurement within 48 hours after the patch was obtained. As a result, tolterodine acetate was detected in the patch obtained in Example 2, which indicated that tolterodine acetate was formed in the adhesive agent layer. On the other hand, no tolterodine acetate was detected in the patches obtained in Comparative Examples 1 to 3, which indicated that tolterodine acetate was not formed.

From the results of the evaluation of tolterodine acetate formation, it was shown that, when tolterodine and the (meth) acrylate copolymer containing vinyl acetate as a constituent were contained in the adhesive agent layer (Example 2), tolterodine acetate was formed over time. Moreover, as is apparent from the results shown in Table 1, it was shown that the patch of the present invention (Example 1) comprising tolterodine acetate in the adhesive agent layer had a strong adhesion force. On the other hand, the patch (Comparative Example 1) comprising no tolterodine acetate in the adhesive agent layer had an inferior adhesion force.

### [Industrial Applicability]

As described above, the present invention makes it possible to provide a patch having a strong adhesion force to the skin, and a method for enhancing adhesion force of a patch.

Hence, the patch and the method for enhancing adhesion force of a patch of the present invention are extremely useful in the pharmaceutical and medical industries.

## Claims

1. A patch comprising a support and an adhesive agent layer comprising a (meth)acrylate copolymer containing vinyl acetate as a constituent and at least one compound selected from the group consisting of (R)-N,N-diisopropyl-3-(2-ethanoyloxy-5-methylphenyl)-3-phenylpropanamine (tolterodine acetate) and salts thereof, wherein the total content of the tolterodine acetate and the salts thereof is 0.05 to 10% by mass relative to the total mass of the adhesive agent layer.

2. The patch according to claim 1, wherein
the adhesive agent layer further comprises at least one compound selected from the group consisting of tolterodine and salts thereof.

3. The patch according to claim 1, wherein
the content of the (meth)acrylate copolymer containing vinyl acetate as a constituent is 5 to 95% by mass relative to the total mass of the adhesive agent layer.

4. A patch comprising a support and an adhesive agent layer, obtainable by a method comprising
allowing the adhesive agent layer, comprising at least one compound selected from the group consisting of tolterodine and salts thereof in an amount of 15 to 45% by mass relative to the total mass of the adhesive agent layer and a (meth)acrylate copolymer containing vinyl acetate as a constituent, to stand at a temperature of 20 to 80°C for 7 days to a half year; and
wherein the total content of the (R)-N,N-diisopropyl-3-(2-ethanoyloxy-5-methylphenyl)-3-phenylpropanamine (tolterodine acetate) and the salts thereof derived in the adhesive layer from the at least one compound selected from the group consisting of tolterodine and salts thereof is 0.05 to 10% by mass relative to the total mass of the adhesive agent layer.

5. The patch according to claim 4, wherein
the content of the (meth)acrylate copolymer containing vinyl acetate as a constituent is 5 to 95% by mass relative to the total mass of the adhesive agent layer.

6. A method for enhancing adhesion force of a patch comprising a support and an adhesive agent layer, the method comprising causing a (meth)acrylate copolymer containing vinyl acetate as a constituent and at least one compound selected from the group consisting of (R)-N,N-diisopropyl-3-(2-ethanoyloxy-5-methylphenyl)-3-phenylpropan-amine (tolterodine acetate) and salts thereof to be present in the adhesive agent layer such that the total content of the tolterodine acetate and the salts thereof is 0.05 to 10% by mass relative to the total mass of the adhesive agent layer.

## Patentansprüche

1. Pflaster, umfassend einen Träger und eine Klebemittelschicht, umfassend ein (Meth)acrylat-Copolymer, enthaltend Vinylacetat als Bestandteil und mindestens eine Verbindung, ausgewählt aus der Gruppe bestehend aus (R)-N,N-Diisopropyl-3-(2-ethanoyloxy-5-methylphenyl)-3-phenylpropanamin (Tolterodinacetat) und Salzen davon, wobei der Gesamtgehalt des Tolterodinacetats und der Salze davon 0,05 bis 10 Massen-%, bezogen auf die Gesamtmasse der Klebemittelschicht, beträgt.

2. Pflaster nach Anspruch 1, wobei die Klebemittelschicht ferner mindestens eine Verbindung, ausgewählt aus der Gruppe bestehend aus Tolterodin und Salzen davon, umfasst.

3. Pflaster nach Anspruch 1, wobei der Gehalt des (Meth)acrylat-Copolymers, enthaltend Vinylacetat als Bestandteil, 5 bis 95 Massen-%, bezogen auf die Gesamtmasse der Klebemittelschicht, beträgt.

4. Pflaster, umfassend einen Träger und eine Klebemittelschicht, erhältlich durch ein Verfahren, umfassend
Stehenlassen der Klebemittelschicht, umfassend mindestens eine Verbindung, ausgewählt aus der Gruppe bestehend aus Tolterodin und Salzen davon in einer Menge von 15 bis 45 Massen-%, bezogen auf die Gesamtmasse der Klebemittelschicht, und einem (Meth)acrylat-Copolymer, enthaltend Vinylacetat als Bestandteil, bei einer Temperatur von 20 bis 80°C für 7 Tage bis zu einem halben Jahr; und
wobei der Gesamtgehalt des (R)-N,N-Diisopropyl-3-(2-ethanoyloxy-5-methylphenyl)-3-phenylpropanamins (Tolterodinacetat) und der Salze davon, die sich in der Klebemittelschicht von der mindestens einen Verbindung, ausgewählt aus der Gruppe bestehend aus Tolterodin und Salzen davon, ableiten, 0,05 bis 10 Massen-%, bezogen auf die Gesamtmasse der Klebemittelschicht, beträgt.

5. Pflaster nach Anspruch 4, wobei der Gehalt des (Meth)acrylat-Copolymers, enthaltend Vinylacetat als Bestandteil, 5 bis 95 Massen-%, bezogen auf die Gesamtmasse der Klebemittelschicht, beträgt.

6. Verfahren zum Erhöhen der Haftkraft eines Pflasters, umfassend einen Träger und eine Klebemittelschicht, wobei das Verfahren umfasst, dass ein (Meth)acrylat-Copolymer, enthaltend Vinylacetat als Bestandteil, und mindestens eine Verbindung, ausgewählt aus der Gruppe bestehend aus (R)-N,N-Diisopropyl-3-(2-ethanoyloxy-5-methylphenyl)-3-phenylpropanamin (Tolterodinacetat) und Salzen davon, in der Klebemittelschicht so vorliegt, dass der Gesamtgehalt des Tolterodinacetats und der Salze davon 0,05 bis 10 Massen-%, bezogen auf die Gesamtmasse der Klebemittelschicht, beträgt.

## Revendications

1. Timbre comprenant un support et une couche d'agent adhésif comprenant un copolymère (méth)acrylate contenant de l'acétate de vinyle comme constituant et au moins un composé sélectionné dans le groupe constitué de la (R) - N,N-diisopropyl-3-(2-éthanoyloxy-5-méthylphényl)-3-phénylpropanamine (acétate de toltérodine) et ses sels, la teneur totale de l'acétate de toltérodine et ses sels étant de 0,05 à 10 % en masse par rapport à la masse totale de la couche d'agent adhésif.

2. Timbre selon la revendication 1,
la couche d'agent adhésif comprenant en outre au moins un composé sélectionné dans le groupe constitué de la toltérodine et ses sels.

3. Timbre selon la revendication 1,
la teneur du copolymère (méth)acrylate contenant de l'acétate de vinyle comme constituant étant de 5 à 95 % en masse par rapport à la masse totale de la couche d'agent adhésif.

4. Timbre comprenant un support et une couche d'agent adhésif, pouvant être obtenu par un procédé comprenant
le fait de permettre à la couche d'agent adhésif, comprenant au moins un composé sélectionné dans le groupe constitué de la toltérodine et de ses sels en une quantité de 15 à 45 % en masse par rapport à la masse totale de la couche d'agent adhésif et à un copolymère (méth)acrylate contenant de l'acétate de vinyle comme constituant, de reposer à une température de 20 à 80°C durant 7 jours à une demi-année ; et
la teneur totale de la (R)-*N*,*N*-diisopropyl-3-(2-éthanoyloxy-5-méthylphényl)-3-phénylpropanamine (acétate de toltérodine) et ses sels dérivés dans la couche adhésive à partir du au moins un composé sélectionné dans le groupe constitué de la toltérodine et ses sels étant de 0,05 à 10 % en masse par rapport à la masse totale de la couche d'agent adhésif.

5. Timbre selon la revendication 4,
la teneur du copolymère (méth)acrylate contenant de l'acétate de vinyle comme constituant étant de 5 à 95 % en masse par rapport à la masse totale de la couche d'agent adhésif.

6. Procédé d'amélioration de la force d'adhésion d'un timbre comprenant un support et une couche d'agent adhésif, le procédé comprenant le fait d'amener un copolymère (méth)acrylate contenant de l'acétate de vinyle comme constituant et au moins un composé sélectionné dans le groupe constitué de la (R)-*N*,*N-*diisopropyl-3-(2-éthanoyloxy-5-méthylphényl)-3-phénylpropanamine (acétate de toltérodine) et ses sels à être présent dans la couche d'agent adhésif de sorte que la teneur totale de l'acétate de toltérodine et ses sels est de 0,05 à 10 % en masse par rapport à la masse totale de la couche d'agent adhésif.
